# DEMANDE DE BREVET EUROPEEN

(11) **EP 1 138 771 A1**
(43) Date de publication de la demande: **04.10.2001**
(21) Numéro de dépôt: 00201175.7
(22) Date de dépôt: 30.03.2000
(51) Int. Cl.: C12N 15/56, C12N 9/24, C12Q 1/68, A23F 5/16, A61K 38/47, A01H 5/00

(54) **Endo-Mannanase de café**

(71) Demandeur: SOCIETE DES PRODUITS NESTLE S.A., 1800 Vevey (CH)
(72) Inventeur: Marraccini, Pierre, 37510 Savonnières (FR); Rogers, John, 37540 St. Cyr-sur-Loire (FR); Pridmore, Raymond David, CH-1000 Lausanne 26 (CH)

(57) **Abrégé**

Fragment d'ADN issu de café codant pour au moins une enzyme impliquée dans l'hydrolyse de polysaccharides constitués au moins de molécules de mannanes pures ou ramifiée liées entre elles par un liaison β (1->4), présentant la séquence nucléique SEQ ID NO: 1 ou étant homologue ou s'hybridant à un fragment d'ADN présentant la séquence nucléique SEQ ID NO: 1.

LISTE DE SEQUENCES

<110> SOCIETE DES PRODUITS NESTLE S.A.

<120> MANNANASE DE CAFE

<130> mannanase II

<140>

<141>

<160> 13

<170> PatentIn Ver. 2.1

<210> 1

<211> 1486

<212> ADN

<213> Coffea arabica

<400> 1

gtgaccagac tctagttcga agcaacaaat taatagctat atcaatcact caatataatc 60

aatgatgtcc agagaaaaga gtctcttgtt aaggtgctgt tctctttcct tggctctttt 120

cattcttctc ggtgttggag agggacatgg tgaaattgcg agtaatagta ccagcagcag 180

cagcttcagc tttgtcaaaa ctaggggaac tgagtttgtg atgaatggga ggccattata 240

cctcaacggc ttcaatgcat attggttaat gtacatggca tctgatccat ctacgaggac 300

gaaggtatca accaccttcc aacaagcttc caagtatgga atgaatgcag ccagaacttg 360

ggctttcagc gacggtggct atagggcttt gcaacaatct cctggttcct acaacgagga 420

catgttcaaa ggtttggatt tcgtagtttc agaagcaaag aagtacggga tccatctcat 480

actcactttg gtcaacaact gggaaggtta cgggggaaag aaacaatatg tccagtgggc 540

gagagatcaa ggacactact tgaacaatga tgatgacttt tttaccgatc caattgtcag 600

aggctacttc aaaaaccaca tcaagactgt tctcacaaga atcaactcca taacggactt 660

tgcatacaaa gacgatccga ccatatttgc atgggagcta atgaatgaac ctcgttgcca 720

aagtgaccta tctggaaaag ctattcagga ttggatctca gaaatggcaa ctcatgtcaa 780

gtccatcgat agcgatcacc tcctagacat tggtcttgaa ggattttatg gagagtctgt 840

gccccaaaag aaggaatata atcctggtta ccaagttggg actgacttta tttccaataa 900

tcgcatagta caagtggatt ttgccaccat tcatttgtat cctgaccaat gggtacccaa 960

ttcgaatgat gagactcaag cacaatttgt ggatagatgg atcaaagagc acatagatga 1020

ttccaaatat ttgctcgaga agccacttct gttgaccgaa ttcggcaagt cttcaagatc 1080

acctgggtac caagtcgcca aaagggatgc gtatttatca catatatacg ataccatcta 1140

cgcttgtgca gcaactcgtg gcggcggcgt atgtggtggt aacctctttt ggcaagtcat 1200

ggctccaggg atggaaagtt ggggcgatgg atatgagatt gtcttggaag agaacccttc 1260

cactgtagga gtaattgctc aacaatccaa caggctatca tctcttacct aaatatggtt 1320
ggcaccaaat ctcaatgatg ttaagagcct actaagaatt catactacaa attctgaaaa 1380
taaaacagtt tttctaggtc ttatgtgaca ttattgtatc aattattaat ttgatactta 1440
aagtatcaat tcataacgag ttattacccg tgtatttgca cattca 1486

<210> 2
<211> 416
<212> PRT
<213> Coffea arabica
<400> 2
Met Met Ser Arg Glu Lys Ser Leu Leu Leu Arg Cys Cys Ser Leu Ser
1               5                   10                  15

Leu Ala Leu Phe Ile Leu Leu Gly Val Gly Glu Gly His Gly Glu Ile
            20                  25                  30

Ala Ser Asn Ser Thr Ser Ser Ser Ser Phe Ser Phe Val Lys Thr Arg
        35                  40                  45

Gly Thr Glu Phe Val Met Asn Gly Arg Pro Leu Tyr Leu Asn Gly Phe
    50                  55                  60

Asn Ala Tyr Trp Leu Met Tyr Met Ala Ser Asp Pro Ser Thr Arg Thr
65                  70                  75                  80

Lys Val Ser Thr Thr Phe Gln Gln Ala Ser Lys Tyr Gly Met Asn Ala
                85                  90                  95

Ala Arg Thr Trp Ala Phe Ser Asp Gly Gly Tyr Arg Ala Leu Gln Gln
            100                 105                 110

Ser Pro Gly Ser Tyr Asn Glu Asp Met Phe Lys Gly Leu Asp Phe Val
        115                 120                 125

Val Ser Glu Ala Lys Lys Tyr Gly Ile His Leu Ile Leu Thr Leu Val
    130                 135                 140

Asn Asn Trp Glu Gly Tyr Gly Gly Lys Lys Gln Tyr Val Gln Trp Ala
145                 150                 155                 160

Arg Asp Gln Gly His Tyr Leu Asn Asn Asp Asp Asp Phe Phe Thr Asp
                165                 170                 175

Pro Ile Val Arg Gly Tyr Phe Lys Asn His Ile Lys Thr Val Leu Thr
            180                 185                 190

Arg Ile Asn Ser Ile Thr Gly Leu Ala Tyr Lys Asp Asp Pro Thr Ile
        195                 200                 205

Phe Ala Trp Glu Leu Met Asn Glu Pro Arg Cys Gln Ser Asp Leu Ser
    210                 215                 220

Gly Lys Ala Ile Gln Asp Trp Ile Ser Glu Met Ala Thr His Val Lys
225                 230                 235                 240

Ser Ile Asp Ser Asp His Leu Leu Asp Ile Gly Leu Glu Gly Phe Tyr
                245                 250                 255

Gly Glu Ser Val Pro Gln Lys Lys Glu Tyr Asn Pro Gly Tyr Gln Val
            260                 265                 270

Gly Thr Asp Phe Ile Ser Asn Asn Arg Ile Val Gln Val Asp Phe Ala
        275                 280                 285

Thr Ile His Leu Tyr Pro Asp Gln Trp Val Pro Asn Ser Asn Asp Glu
    290                 295                 300

Thr Gln Ala Gln Phe Val Asp Arg Trp Ile Lys Glu His Ile Asp Asp
305                 310                 315                 320

Ser Lys Tyr Leu Leu Glu Lys Pro Leu Leu Leu Thr Glu Phe Gly Lys
                325                 330                 335

Ser Ser Arg Ser Pro Gly Tyr Gln Val Ala Lys Arg Asp Ala Tyr Leu
            340                 345                 350

Ser His Ile Tyr Asp Thr Ile Tyr Ala Cys Ala Ala Thr Arg Gly Gly
        355                 360                 365

Gly Val Cys Gly Gly Asn Leu Phe Trp Gln Val Met Ala Pro Gly Met
    370                 375                 380

Glu Ser Trp Gly Asp Gly Tyr Glu Ile Val Leu Glu Glu Asn Pro Ser
385                 390                 395                 400

Thr Val Gly Val Ile Ala Gln Gln Ser Asn Arg Leu Ser Ser Leu Thr
                405                 410                 415

<210> 3
<211> 22
<212> PRT
<213> Coffea arabica
<400> 3
Ser Phe Ser Phe Val Lys Thr Arg Gly Thr Glu Phe Val Met Asn Gly
1               5                   10                  15

Arg Pro Leu Tyr Leu Asn
            20

<210> 4
<211> 10
<212> PRT
<213> Coffea arabica
<400> 4
Thr Trp Ala Phe Ser Asp Gly Gly Tyr Arg
1               5                   10

<210> 5
<211> 17
<212> PRT
<213> Coffea arabica
<400> 5
Glu Tyr Asn Pro Gly Tyr Gln Val Gly Thr Asp Phe Ile Ser Asn Asn
1               5                   10                  15
Arg

<210> 6
<211> 18
<212> ADN
<213> Séquence artificielle
<220>
<223> Description de la séquence artificielle:NUCLEOTIDE
      DE SYNTHESE MAN 202
<400> 6
ggnacngart tygtnatg                                                18

<210> 7
<211> 17
<212> ADN
<213> Séquence artificielle
<220>
<223> Description de la séquence artificielle:NUCLEOTIDE
      DE SYNTHESE MAN 203
<400> 7
ccrtcrctra angccca                                                 17

<210> 8
<211> 19

<212> ADN
<213> Séquence artificielle
<220>
<223> Description de la séquence artificielle:NUCLEOTIDE
      DE SYNTHESE MAN 214
<400> 8
atcaaccacc ttccaacaa                                                    19

<210> 9
<211> 18
<212> ADN
<213> Séquence artificielle
<220>
<223> Description de la séquence artificielle:NUCLEOTIDE
      DE SYNTHESE MAN 215
<400> 9
taccttcgtc ctcgtaga                                                     18

<210> 10
<211> 20
<212> ADN
<213> Séquence artificielle
<220>
<223> Description de la séquence artificielle:NUCLEOTIDE
      DE SYNTHESE T3
<400> 10
aattaaccct cactaaaggg                                                   20

<210> 11
<211> 22
<212> ADN
<213> Séquence artificielle
<220>

<223> Description de la séquence artificielle:NUCLEOTIDE
      DE SYNTHESE T7
<400> 11
gtaatacgac tcactatagg gc                                              22

<210> 12
<211> 21
<212> ADN
<213> Séquence artificielle
<220>
<223> Description de la séquence artificielle:NUCLEOTIDE
      DE SYNTHESE MAN 300
<400> 12
gtgaccagac tctagttcga a                                               21

<210> 13
<211> 22
<212> ADN
<213> Séquence artificielle
<220>
<223> Description de la séquence artificielle:NUCLEOTIDE
      DE SYNTHESE MAN 301
<400> 13
tgaatgtgca aatacacggg ta                                              22

## Description

### Endo-Mannanase de café

La présente invention se rapporte à l'utilisation de fragments d'ADN de café codant pour au moins une enzyme impliquée dans l'hydrolyse des polysaccharides constitués au moins de molécules de mannanes simples ou ramifiées et liées entre elles par une liaison β (1->4).

### Etat de la technique

Les polysaccharides qui contiennent du mannose sont fréquemment présents dans les parois cellulaires des végétaux supérieurs en particulier chez les légumineuses et sont considérés comme une réserve de carbohydrates dans les graines.

Dans plusieurs plantes, il a été montré que l'activité endo-β-mannanase est principalement détectée dans l'endosperme des graines en cours de germination (Bewley, Trends Plant Sci 2, 464-469, 1997).

Dans le grain de café, on trouve notamment des galactomannanes. Ces dernières représentent environ 24% du poids sec du grain (Bradbury and Halliday, J Agric Food Chem 38, 389-392, 1990). Ces polysaccharides sont formés d'une chaîne linéaire de résidus mannosyl qui sont liés entre eux par des liaisons de type β-1->4 et sur laquelle sont fixés des monomères de résidus α-galactosyl. Il est également connu que l'enzyme appelée endo-β-mannanase (E.C 3.2.1.78) est une hydrolase qui dégrade les polymères de (1->4)- β -mannanes, ainsi facilitant la sortie de la radicelle pendant la germination et libérant des petits oligosaccharides qui sont utilisés ensuite comme source d'énergie pour la croissance de la jeune plante.

Dans les procédés industriels, lors du traitement du café, les molécules de mannanes et leurs dérivés constituent une partie importante des sédiments insolubles. De plus, la fraction de ces molécules qui entre en solution lors de la première extraction (environ 50 %) est aussi très faiblement soluble, et est donc responsable pour la majorité des précipitations secondaires se manifestant pendant les étapes suivantes. Dans le brevet EP 0676145A, il a été démontré qu'il est possible d'hydrolyser les galactomannanes de café en utilisant une mannanase immobilisée extraite d'*Aspergillus niger*.

La demande EP n° 98203742.6 propose, pour sa part, l'utilisation de fragments d'ADN de café codant pour au au moins une endo-β-mannanase impliquée dans l'hydrolyse des polysaccharides constitués au moins de molécules de mannanes simples ou ramifiées et liées entre elles par une liaison β (1->4).

### Il est cependant apparu intéressant d'isoler encore d'autres enzymes et gènes issus de la graine café.

### Résumé de l'invention

A cet effet la présente invention a pour objet tout fragment d'ADN issu du café codant pour au moins une enzyme impliquée dans l'hydrolyse de tels polysaccharides, présentant la séquence nucléique SEQ ID NO: 1 ou étant homologue ou s'hybridant à un fragment d'ADN présentant la séquence nucléique SEQ ID NO: 1.

La présente invention concerne également l'utilisation de tout ou partie de tels fragments d'ADN comme amorce pour faire une PCR ou comme sonde pour détecter *in vitro* ou modifier *in vivo* au moins un gène de café codant pour au moins une endo-β-mannanase.

La présente invention concerne également toute protéine issue de la graine de café codée par un tel gène de café et impliquée dans l'hydrolyse des polysaccharides constitués au moins de molécules de mannanes simples ou ramifiées et liées entre elles par une liaison β (1->4) et ayant la séquence en acides aminés SEQ ID NO: 2 ou toute séquence en acides aminés homologue à cette dernière.

Un autre objet de l'invention concerne tout micro-organisme et de toute cellule végétale comprenant, intégré dans son génome ou par le moyen d'un plasmide replicable, un fragment d'ADN selon la présente invention.

Enfin l'invention concerne une composition alimentaire, cosmétique ou pharmaceutique comprenant un fragment d'ADN ou une protéine selon l'invention.

### Description des figures

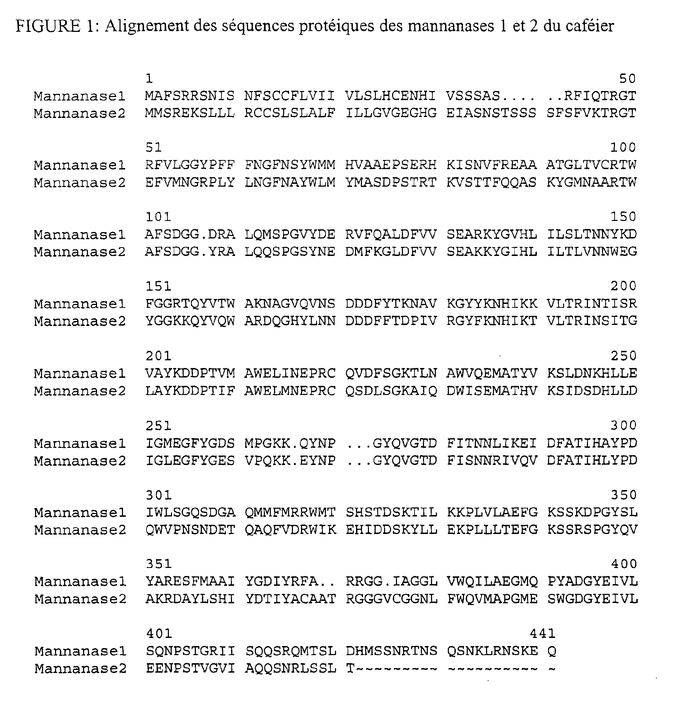

La figure 1 représente un alignement protéique des endo-β-mannanase d'*Aspergillus aculeatus*, de *Trichoderma reesei*, de *Lycopersicon esculentum* (tomate) avec la mannanase 1 (P. Marraccini et J.Rogers, EP N° 98203742.6) et la mannanase du caféier selon la présente invention.

### Description détaillée de l'invention

Au sens de la présente invention, on entend par "séquence homologue" toute séquence nucléique ou d'acides aminés ayant une fonction identique, ne différant des séquences selon l'invention que par la substitution, la délétion ou l'addition d'un petit nombre de bases nucléiques ou d'acides aminés, par exemple 1 à 500 paires de bases (pb) ou 1 à 150 acides aminés.

Dans ce cadre, on considérera en particulier comme homologues deux séquences d'ADN qui, du fait de la dégénérescence du code génétique, codent pour un même polypeptide. De même, on considérera comme homologues deux protéines fonctionnelles qui sont reconnues par un même anticorps, le rapport des valeurs d'intensité de reconnaissance des deux protéines par l'anticorps n'excédant pas 100, par exemple.

On considérera aussi comme séquence homologue, celle qui présente plus de 70% d'homologie avec les séquences selon l'invention, en particulier plus de 80% ou 90%. Dans ce dernier cas, l'homologie est déterminée par le rapport entre le nombre de bases ou d'acides aminés d'une séquence homologue qui sont identiques à celles d'une séquence selon l'invention, et le nombre total de bases ou d'acides aminés de ladite séquence selon l'invention.

Au sens de la présente invention, on entend par "fragment qui s'hybride" tout fragment capable de s'hybrider aux fragments selon l'invention par la méthode de Southern-Blot (Sambrook *et al*., Molecular Cloning, A Laboratory Manual, Cold Spring Harbor Laboratory Press, U.S.A., 1989, chapitres 9.31 à 9.58). De préférence, l'hybridation est conduite dans des conditions stringentes de manière à éviter des hybridations aspécifiques ou peu stables.

Enfin, le terme "fragment" ou "fragment d'ADN" doit être compris comme un ADN double brin d'origine chromosomique, qui peut être synthétisé, reproduit *in-vitro* par exemple par la méthode connue appelée "Polymérase Chain Reaction", ou reproduit *in-vivo* dans une bactérie du type *Escherichia coli,* par exemple.

Dans la suite de la description, les séquences SEQ ID NO: font référence aux séquences présentées dans la liste des séquences ci-après. Les oligonucléotides de synthèse SEQ ID NO: 6 à SEQ ID NO: 13 mentionnés dans la description et présentés dans la liste des séquences ci-après, sont fournis par Eurogentec (Parc Scientifique du Sart Tilman-4102 Seraing-Belgium).

On a pu montrer que tout ou partie de la séquence SEQ ID NO: 1 permet, suite à une transformation, d'hydrolyser des polysaccharides constitués au moins de molécules de mannanes pures ou ramifiées liées entre elles par une liaison β (1->4) dans une cellule hôte, comme une cellule de plante ou un micro-organisme.

La présente invention concerne tout fragment d'ADN ayant la séquence nucléique SEQ ID NO: 1 ou tout fragment d'ADN homologue ou s'hybridant à cette séquence nucléique codant pour au moins enzyme impliquée dans l'hydrolyse de polysaccharides constitués au moins de molécules de mannanes pures ou ramifiée liées entre elles par un liaison β (1->4).

L'enzyme est de préférence une endo-β-mannanase ayant la séquence en acides aminés SEQ ID NO: 2, ou toute séquence en acides aminés homologue à cette dernière. L' endo-β-mannanase pouvant contenir au moins une des séquences en acides aminés suivantes: SEQ ID NO: 3 à 5.

De préférence, l'invention concerne le fragment d'ADN délimité par les nucléotides 62 à 1312 de la séquence nucléique SEQ ID NO: 1.

La présente invention concerne également les nouvelles enzymes codées par les gènes de la séquence SEQ ID NO: 1, notamment les séquences qui leur sont homologues. On peut ainsi envisager de les utiliser pour modifier ou dégrader *in-vitro* de tels polysaccharides, par exemple. Pour cela, il est préférable de purifier au moins une de ces enzymes, en surexprimant classiquement leur gène dans une bactérie et en les isolant classiquement, par précipitation et/ou chromatographie du milieu de culture, par exemple.

L'invention concerne également l'utilisation de tout ou partie de fragments d'ADN selon l'invention, notamment comme amorce pour faire une PCR ou comme sonde pour détecter *in vitro* ou modifier *in vivo* au moins un gène de café codant pour au moins une endo-β-mannanase. On utilise de préférence au moins 10 paires de base.

Par ailleurs, la présente invention a pour objet une protéine issue de la graine de café codée par un gène de café et impliquée dans l'hydrolyse de polysaccharides constitués au moins de molécules de mannanes pures ou ramifiés liés entre elles par une liaison β (1->4) et ayant la séquence en acides aminés SEQ ID NO: 2 ou toute séquence en acides aminés homologue à cette dernière. L' endo-β-mannanase pouvant contenir au moins une des séquences en acides aminés suivantes: SEQ ID NO: 3 à 5.

Un autre objet de la présente invention concerne un procédé d'hydrolyse de polysaccharides constitués au moins de molécules de mannanes pures ou ramifiées liées entre elles par une liaison β (1->4), dans lequel (1) on clone dans un vecteur un fragment d'ADN codant pour l'enzymes selon l'invention, ledit vecteur comprenant en outre une séquence permettant la réplication autonome ou l'intégration dans une cellule hôte, (2) on transforme une cellule hôte par ledit vecteur, (3) puis on cultive la cellule hôte transformée dans des conditions appropriées pour l'hydrolyse de tels polysaccharides.

La présente invention ouvre donc la possibilité d'utiliser des fragments d'ADN selon l'invention pour modifier la production de polysaccharides constitués au moins de molécules de mannanes pures ou ramifiées liées entre elles par une liaison β (1->4) dans un cellule hôte, notamment une cellule de grain de café. On peut ainsi envisager d'exprimer ou de surexprimer dans une cellule de grain de café l'expression des ADN selon l'invention, pour produire de tels polysaccharides destinés à modifier l'arôme et la structure des grains de café, par exemple.

Enfin, la présente invention fournit aussi de nouvelles enzymes impliquées dans l'hydrolyse de tels polysaccharides. Ces enzymes peuvent être ainsi avantageusement utilisées pour synthétiser ou modifier *in-vitro* de tels polysaccharides.

La présente invention concerne également une cellule végétale comprenant, intégré dans son génome ou par le moyen d'un vecteur recombinant, un fragment d'ADN ayant la séquence nucléotidique SEQ ID NO: 1 ou un fragment d'ADN ayant une séquence nucléique homologue ou qui s'hybride à la séquence nucléique SEQ ID NO: 1 ou un fragment d'ADN comprenant au moins les nucléotides 62 à 1312 de la séquence nucléique SEQ ID NO: 1.

La cellule végétale est de préférence une cellule de café. On peut notamment choisir comme cellules de café des cellules issues de plante de *Coffea canephora* var. robusta, *Coffea arabica* ou toute autre espèce du genre *Coffea*.

La présente invention concerne également toute plante ou toute graine constituées de cellules végétales comprenant, intégré dans son génome ou par le moyen d'un vecteur recombinant, un fragment d'ADN ayant la séquence nucléotidique SEQ ID NO: 1 ou un fragment d'ADN ayant une séquence nucléique homologue ou qui s'hybride à la séquence nucléique SEQ ID NO: 1 ou un fragment d'ADN comprenant au moins les nucléotides 62 à 1312 de la séquence nucléique SEQ ID NO: 1.

Tout micro-organisme comprenant, intégré dans son génome ou par le moyen d'un plasmide replicable, un fragment d'ADN selon l'invention, de manière à ce qu'il exprime au moins une enzyme impliquée dans l'hydrolyse de polysaccharides constitués au moins de molécules de mannanes pures ou ramifiés liés entre elles par une liaison β (1->4), est également un objet de la présente invention.

Un autre objet de l'invention concerne toute composition alimentaire, cosmétique ou pharmaceutique comprenant un fragment d'ADN selon l'invention ou une protéine selon l'invention.

Enfin, la présente invention concerne un procédé de traitement de grains de café, dans lequel on utilise toute ou partie de la protéine selon l'invention. On peut notamment utiliser toute ou partie de la protéine selon l'invention pour augmenter le pourcentage de matière sèche extraite, lors du traitement de grains de café. En utilisant toute ou partie de la protéine selon l'invention, on peut ainsi augmenter le rendement d'extraction tout en diminuant la quantité de sédiments.

Après surexpression du fragment d'ADN selon l'invention dans un micro-organisme, dans un champignon ou dans une cellule végétale indifférenciée, on peut traiter les sédiments avec l'enzyme plus ou moins purifiée, de manière ainsi à augmenter les rendements d'extraction.

Après surexpression du fragment d'ADN selon l'invention dans un micro-organisme, dans un champignon ou dans une cellule végétale indifférenciée, on peut également traiter la liqueur du café, de manière à diminuer la sédimentation dû aux mannanes qui gélifient.

La présente invention est décrite plus en détail ci-après à l'aide du complément de description qui va suivre, qui se réfère à des exemples d'obtention de fragments d'ADN, de plasmides recombinants et de bactéries transformées selon l'invention. Il va de soi, toutefois, que ces exemples sont donnés à titre d'illustration de l'objet de l'invention dont ils ne constituent en aucune manière une limitation. La manipulation de l'ADN, le clonage et la transformation de cellules bactériennes sont, en l'absence de précisions contraires, effectués selon les protocoles décrits dans l'ouvrage de Sambrook *et al*. cité plus haut. Les pourcentages sont donnés en poids, sauf indication contraire.

### Exemple 1

### Mesure du pic d'activité de l'endo-β-mannanase durant la germination

Des grains de la variété *Coffea arabica* var. caturra 2308 sont récoltés au stade mature, dépulpés et séchés pendant trois jours à la température ambiante. Ces grains sont ensuite déparchés, séchés puis stérilisés pour être mis en germination en culture *in-vitro*. Pour ce faire, ils sont placés 1 heure dans du Rovral (0.12 % v/v), rincés à l'eau stérile, placés 1 heure dans une solution d'hypochlorite de calcium (6 % p/v) à laquelle on ajoute quelques gouttes d'émulsifiant Teepol, puis sont rincés par 4 passages à l'eau stérile avant d'être mis en culture dans des tubes à essai sur un milieu agar-eau. La germination se déroule à 25°C en présence de lumière. Le moment où les grains sont mis sur le lit d'agar est considéré comme jour après imbibition zéro (JAI = 0).

Les lots de grains sont ensuite récoltés à différents stades de germination (JAI 7, 14, 21, 28) puis sont broyés dans l'azote liquide. Ensuite la poudre est homogénéisée à raison de 1 g pour 5 ml dans un tampon d'extraction (phosphate-citrate 200/100 mM pH 5.0, métabisulfite Na₂S₂O₅ 10 mM, EDTA 5 mM, inhibiteur de protéase 'Complete' [cat. n° 1836 145, Boehringer Mannheim, Mannheim, Allemagne] 1 comprimé / 50 ml) pendant 20 min. à 4°C. L'homogénat est ensuite centrifugé à 12000 g pendant 20 min. à 4°C, et le surnageant est repris et centrifugé une deuxième fois. Le surnageant, correspondant à l'extrait enzymatique brut, est ensuite aliquoté et congelé à -80°C.

L'activité enzymatique de l'endo-β-mannanase est dosée selon la méthode suivante. Un extrait enzymatique brut de 400 µl est ajouté à 1.6 ml de tampon de réaction (NaCl 100 mM, acétate de sodium 200 mM pH 5.0) contenant du substrat insoluble (AZCL-Galactomannane, Megazyme, Co. Wicklow, Ireland) en quantité finale de 1 % p/v. La réaction débute par l'ajout de l'extrait, et se déroule à 37°C sous agitation. Pour calculer la pente initiale de la réaction, un aliquote de 400 µl de milieu est prélevé toutes les 15 min. pendant 1 h, chauffé à 100°C pendant 5 min. puis centrifugé à 12000 *g* pendant 2 min. La mesure de la densité optique du surnageant se fait à 590 nm et l'activité spécifique est exprimée en UA (unités d'absorption optique) min⁻¹ mg protéine⁻¹, après avoir dosé la concentration protéique dans chaque extrait par la méthode de Bradford (Bradford, Anal. Biochem. 72, 248-254, 1976). Ainsi on trouve que l'activité est quasiment nulle pendant les 14 premiers jours après imbibition (JAI), et augmente après progressivement jusqu'à un pic maximum autour de 28 JAI. Après 28 JAI, l'activité baisse lentement.

### Exemple 2

### Etapes de purification de l'endo-β-mannanase

Selon les résultats décrits précédemment, la stratégie de purification se poursuit en utilisant 16 ml d'un extrait d'enzyme brut de 28 JAI ayant une activité autour de 0.2 UA, min⁻¹. mg protéine⁻¹ x 10⁻², un contenu de protéines totales d'environ 48 mg, et une activité totale de 9.6 UA min⁻¹ x 10⁻².

### 1. Précipitation au sulfate d'ammonium:

Dans un premier temps, l'extrait enzymatique brut est fractionné par une précipitation au sulfate d'ammonium à 4°C. Le sulfate d'ammonium est ajouté lentement sous agitation jusqu'à un niveau de saturation de 35 % et la solution est ensuite centrifugée à 12000 g à 4°C pendant 20 min. Le culot ainsi obtenu est repris dans un minimum (1 ml) de tampon d'extraction (voir ci-dessus). Dans cet extrait, la concentration protéique est approximativement 10 mg. ml⁻¹. L'activité spécifique endo-β-mannanase est de 0.9 UA min⁻¹. mg⁻¹ x 10⁻², ce qui correspond à un enrichissement de l'enzyme d'un facteur 4 par rapport à l'extrait brut et une récupération de 10 UA min⁻¹ de l'activité totale, soit 100 %.

### 2. Séparation sur colonne d'interaction hydrophobe

L'échantillon décrit ci-dessus est ensuite séparé sur une colonne d'interaction hydrophobe (Hiload HR 16/10 phényl sépharose High performance, Amersham Pharmacia Biotech, Suède). La colonne est préalablement équilibrée avec un tampon d'équilibration (phosphate de sodium 50 mM, 400 mM sulfate d'ammonium, pH 7.0). L'échantillon (1 ml) est ensuite injecté sur la colonne qui est ensuite lavée avec 5 volumes de colonne du tampon d'équilibration. Un gradient de 0 à 99.5 % d'eau en 0.5 volumes de colonne est appliqué suivit d'un autre de 99.5 à 100 % en 5 volumes de colonne. L'activité est concentrée principalement dans trois fractions qui sont utilisées pour continuer la purification. Le rendement de purification de cette étape est autour de 80 % par rapport à l'étape précédente. L'activité spécifique est de 27 UA min⁻¹. mg⁻¹, soit un enrichissement d'environ 137 fois par rapport à l'extrait brut. L'activité totale récupérée est d'environ 9 UA min⁻¹ x 10⁻² soit 90 % de l'activité initiale.

### 3. Séparation par chromatographie échangeuse d'ions

Les trois fractions décrites ci-dessus sont mélangées, concentrées et changées de tampon grâce à des tubes Ultrafree (Millipore, Bedford, MA, USA, centrifugation à 4000 *g* maximum). Le volume récupéré est de 1 ml. Cet échantillon est injecté sur une colonne Resource Q (Amersham Pharmacia Biotech AB SE-751 84 Uppsala Suède) échangeuse d'anions, préalablement équilibrée avec un tampon Tris/HCl 20 mM, pH 8.0. L'élution est réalisée avec un gradient linéaire de 0 à 1 M NaCl en 20 volumes de colonne. L'activité totale récupérée est présente exclusivement dans deux fractions. Le rendement de purification de cette étape est de 58 % par rapport à l'étape précédente. L'activité spécifique est de 167 UA min⁻¹. mg⁻¹, soit un enrichissement d'environ 836 fois par rapport à l'extrait brut. L'activité totale récupérée est 0,9 UA min⁻¹, soit environ 9 % de l'activité initiale.

### 4. Séparation par colonne de filtration sur gel

A partir de la colonne échangeuse d'anions, les deux fractions récupérées sont concentrées par centrifugation à 4000 g à 100 1 en utilisant les tubes Ultrafree (Millipore Co, 80 Ashby Road Bedford, MA, USA). Cet échantillon est injecté sur une colonne Superdex 75 HR 10/30 (Amersham Pharmacia Biotech, Suède) préalablement équilibrée avec un tampon de phosphate de sodium 50 mM, NaCl 150 mM, pH 7.0. Les protéines sont éluées avec le même tampon avec un débit de 0.3 ml. min⁻¹. Une courbe de calibration de la colonne est réalisée dans les mêmes conditions grâce à des standards de poids moléculaire. L'activité endo--mannanase est répartie dans deux fractions, correspondant à un poids moléculaire entre 40 et 55 KDa. Le rendement de purification de cette dernière étape est de 48 %. L'activité spécifique est d'environ 1400 UA min⁻¹. mg⁻¹, soit un enrichissement de 7000 fois par rapport à l'extrait brut. L'activité totale est de 0.45 UA min⁻¹, soit 4.5 % de l'activité initiale.

### 5. Analyses par électrophorèse bi-dimensionelle et micro-séquençage des acides aminés de l'enzyme purifiée

Les fractions possédant l'activité enzymatique en sortie des colonnes décrites précédemment sont analysées au cours de la purification par des électrophorèses bi-dimensionelles. Pour ce faire, les fractions sont mélangées et concentrées jusqu'à 20 µl par une centrifugation dans les tubes Ultrafree (Millipore, USA) comme décrit précédemment. A ce volume, on ajoute 105 l de tampon de réhydratation (urée 8 M, CHAPS 3 % p/v, ampholines 0.8 % v/v, DTT 1 % p/v) et on réhydrate un gel strip de 7 cm non-linéaire (pH 3.0 à 10.0) (Immobiline Dry Strip, Amersham Pharmacia Biotech, Suède), selon les instructions du fabricant. Les protéines sont ensuite séparées en fonction de leur point isoélectrique (pI) en utilisant, par exemple, le système IPGphore (Amersham Pharmacia Biotech, Suède) en employant un nombre total de 14 000 volt-heures.

Suivant la séparation des protéines en fonction de leur pI, elles sont ensuite séparées dans une deuxième dimension selon leur poids moléculaires. Cette séparation est réalisé selon les recommandations de Hochstrasser *et al*. (Anal Biochem, 173, 412-423, 1989) et de Gorg *et al*. (Electrophoresis, 8, 122-124, 1987). Ainsi, le gel strip de la première dimension est équilibré dans une première solution (urée 6 M, glycérol 30 % v/v, SDS 2 % p/v, DTT 2 %, Tris-HCl 50 mM, pH 8.0) pendant 5 min., puis est équilibré dans une deuxième solution (urée 6 M, glycérol 30 % v/v, SDS 2 % p/v, iodoacétamide 2.5 % p/v, Tris-HCl 50 mM, pH 8.0) pendant 10 min. Le gel strip est ensuite chargé dans un gel de gradient de concentration d'acrylamide 10-20 % (dimensions 10 x 10 x 0.75 cm) avec un seul puit, et recouvert d'une solution d'agarose (agarose 1 % p/v, SDS 0.5 % p/v, traces de bleu de bromophénol) préalablement chauffée à 90°C et maintenue à 40°C. Le gel est monté dans un système d'électrophorèse vertical et est soumis à une tension de 170 V pendant 2 h. Après la migration, les protéines sont colorées à l'argent selon la méthode de Bjellqvist *et al*. (Electrophoresis, 14, 1357-1365, 1993). Le profil du mélange des trois dernières fractions ainsi obtenues montre la présence d'un seul groupe de protéines qui consiste en une ligne de 5 protéines ayant le même poids moléculaire approximatif de 42 kDa mais possédant de faibles différences de pI entre pI 5.5 et 6. Cette estimation de pI est confirmée par le fait que l'activité endo-β-mannanase est éluée à partir d'une colonne chromatofocusing (mono P HR 5/5 - Amersham Pharmacia Biotech, Suède), à un pH de 5.7 (résultats non montrés) et aussi par l'estimation du pI théorique (5.8) de la protéine mature (voir ci-dessous).

Les protéines ainsi purifiées sont analysées par une micro-séquençage des acides aminés. Pour ce faire, elles sont transférées sur une membrane PVDF ('Problot', Perkin Elmer-Applied Biosystems, 850 Lincoln Centre Drive, Foster City, CA 94404, USA) en utilisant, par exemple, une cellule de transfert Trans-Blot (Bio-Rad, 2000 Alfred Drive, Hercules, California 94547, USA). Ainsi, suivant la séparation de la deuxième dimension, le gel est récupéré et mis en agitation dans une solution de transfert (méthanol 10 % v/v, CAPS-NaOH 10 mM pH 11.0) durant 10 min. Pendant ce temps deux supports de mousse, deux morceaux de papier Whatman et une membrane PVDF-Problot (Perkin Elmer-Applied Biosystem, USA) sont humidifiés dans le même solution. Le gel, la membrane et les supports sont montés dans le système Trans-Blot, selon les instructions du fabricant (Bio-Rad, USA), et le transfert est réalisé sous un courant de 100 V pendant une heure à une température de 4°C. A la fin du transfert, les protéines transférées sur la membrane sont révélées avec une coloration légère de bleu de Coomassie selon les instructions du fabricant de la membrane Problot. Les différentes protéines sont excisées de la membrane, mélangées et séquencées ensemble. Le séquençage N-terminal des protéines purifiées et des peptides internes est réalisé avec un séquenceur automatique Beckman (Beckmann Instruments Inc., 250 Harbor Boulevard Box 3100, Fullerton, California, 92634 USA) selon les méthodes décrites dans Teixeira *et al*. (Electrophoresis, 18, 1491-1497).

Trois séquences sont obtenues par cette méthode. Une séquence N-terminale de 22 acides aminés, appelée SEQ ID NO: 3, et deux autres concernant des peptides internes indépendants, obtenus par une digestion trypsine, de 10 et 17 acides aminés respectivement décrits dans les séquences SEQ ID NO: 4 et 5.

Toutes les séquences obtenues sont sans ambiguïtés, et montrent que les **3** protéines, qui composent la ligne décrite précédemment, sont des isozymes de l'endo-β-mannanase qui partagent une séquence identique dans les régions analysées.

### Exemple 3

### Isolement de l'ADNc pleine longueur codant pour l'endo-β-mannanase de café purifiée des grains en cours de germination

### 1. Isolement des ARN totaux et des ARN messagers poly A+ à partir du grain de café en germination

Les graines de café (*Coffea arabica* var. caturra 2308) sont récoltées au stade mature et germées *in vitro* comme décrit précédemment.

On extrait les ARN totaux de grains après 22 jours de mise en germination (JAI 22). Pour ce faire, on broie rapidement le grain dans de l'azote liquide et la poudre obtenue est resuspendue dans 8 ml de tampon à pH 8.0 contenant du Tris HCl 100 mM, 0,1% p/v de SDS et 0,5% v/v de β-mercaptoéthanol, on l'homogénéise avec un volume de phénol saturé en Tris HCl 100 mM pH 8.0, puis on centrifuge à 12000 *g* pendant 10 min. à 4°C, de manière à extraire la phase aqueuse que l'on centrifuge (i) une fois avec un volume équivalent de phénol, (ii) deux fois avec un volume équivalent de phénol:chloroforme (1:1) et (iii) deux fois avec un volume équivalent de chloroforme (Rogers *et al*., Plant Physiol. Biochem. 37, 261-272, 1999)

On précipite alors les acides nucléiques totaux pendant 1 h à -20°C en ajoutant à la phase aqueuse 1/10 de volume d'acétate de sodium 3 M, pH 5.2 et 2,5 volumes d'éthanol.

Puis on centrifuge le tout à 12000 g pendant 30 min. à 4°C et l'on reprend le culot dans 10 ml d'H₂O, avant de précipiter à nouveau les acides nucléiques en présence de LiCl (2 M final) et d'éthanol (2,5 volumes).

Après centrifugation, on reprend le culot d'ARN totaux dans 1 ml d'H₂O et on le digère pendant 1 h à 37°C à la DNAse RQ1 (Promega Corporation, 2800 Woods Hollow Road, Madison, Wisconsin 53711 USA), afin d'éliminer toute trace d'ADN, puis, on déprotéinise les ARN totaux par un traitement au phénol et au chloroforme, avant de les précipiter en présence d'acétate de sodium comme décrit ci-dessus.

On reprend alors les ARN totaux dans 500 µl d'H₂O et on les quantifie par dosage spectrophotométrique à 260 nm. Leur qualité est analysée par électrophorèse en gel d'agarose en présence de formaldéhyde.

Pour ce faire, on purifie ensuite les ARN messagers poly A+ (ARNm) à partir de 500 µg d'ARN totaux en utilisant le système de purification Oligotex-dT (Qiagen INC., 9600 De Soto Avenue, Chatsworth, California, 91311 USA), puis on évalue la quantité des ARN messagers à l'aide du kit DNA Dipstick (InVitrogen BV, De Schelp 12, 9351 NV Leek, Netherlands).

### 2. Construction et criblage de la banque d'ADNc

On réalise la synthèse d'ADN complémentaire (ADNc), nécessaire à la construction des banques, selon les recommandations fournies dans le kit "Riboclone cDNA synthesis system M-MLV (H-)" (Promega, USA), hormis l'étape de ligature des adaptateurs *Eco*RI. Ceci permet de cloner ces ADNc directement au site unique Srf*I* du vecteur pPCR-Script Amp SK(+) (Stratagene, 11011 North Torrey Pines Road, La Jolla, California 92037, USA). L'efficacité de cette réaction de synthèse d'ADNc est suivie par l'addition d'alpha-(³²P)-dCTP lors de la synthèse des deux brins d'ADN.

Après migration sur gel d'agarose alcalin (Sambrook *et al*., Molecular Cloning, A Laboratory Manual, Cold Spring Harbor Laboratory Press, U.S.A., 1989), on estime la taille des ADNc néosynthétisés comme variant de 0.2 à plus de 4.3 kb. Les quantifications, à l'aide du kit DNA Dipstick (InVitrogen, Netherlands), montrent qu'environ 100 ng d'ADNc sont synthétisés à partir de 1 µg d'ARNm.

Les ADNc ligaturés dans le vecteur pPCR-Script Amp SK(+) (Stratagene, USA) ont été utilisés pour transformer la souche d'*Escherichia coli* XL2-Blue MRF' (Stratagene, USA). On sélectionne les bactéries qui contiennent des vecteurs recombinants sur boîtes de milieu LB (Luria-Bertani) contenant 100 µg. ml⁻¹ d'ampicilline, et en présence d'IPTG et de X-Gal (Sambrook *et al*., 1989). On extrait ensuite les plasmides de cette banque d'ADNc à partir d'une culture de nuit correspondant à un mélange de transformants, en présence de 25 ml milieu LB contenant 100 µg. ml⁻¹ d'ampicilline, et en utilisant le kit "QiaFilter Plasmid MidiKit" (Qiagen INC., USA).

### 3. Isolement de l'ADNc codant pour l'endo-β-mannanase de café

Cette banque d'ADNc de grains en germination a été testée par PCR en utilisant des oligonucléotides de synthèse déduits du résultat du séquencage N-terminal et interne de la mannanase purifiée.

On utilise les oligonucléotides de synthèse dégénérés MAN 202, ayant la séquence nucléique SEQ ID NO: 6, et l'oligonucléotide de synthèse MAN 203, ayant la séquence nucléique SEQ ID NO: 7. L'oligonucléotide de synthèse MAN 202 correspond aux acides aminés 9 à 14 (GTEFVM) de la séquence N terminale (SEQ ID NO: 3) de la mannanase purifiée. L'oligonucléotide de synthèse MAN 203 correspond à la séquence complémentaire codant pour les acides aminés WAFSDG localisés en position position 2 à 7 du peptide interne de la mannanase purifiée correspondant à la séquence SEQ ID NO: 4

On réalise la réaction de PCR en présence de 10 ng de plasmide de la banque d'ADNc, dans un volume final de 50 µl contenant 50 mM KCl, 10 mM Tris-HCl pH 8.8, 1.5 mM MgCl₂, 0.1 mg. ml⁻¹ gélatine, 0.2 mM de chaque dNTP, 0,25 µM de chaque oligonucléotide (MAN 202 et 203) et 3 unités d'ADN polymérase *Taq* (Stratagene, USA). On utilise l'appareil de PCR Robocycler 96 (Stratagene, USA) muni d'un couvercle chauffant et on incube les réactions pendant 35 cycles (94° C-1 min, 45° C-1 min 30 s, 72° C-2 min) suivi d'une extension finale à 72° C pendant 7 min. A l'issue de cette réaction, on obtient un produit de PCR unique d'environ 170 pb qui a été directement ligaturé dans le vecteur pGEMT-easy, selon les recommandations du fournisseur (Promega, USA). La ligature est ensuite utilisée pour transformer la souche d'*Escherichia coli* XL2-Blue MRF' (Stratagene, USA). On sélectionne les bactéries qui contiennent des vecteurs recombinants, sur boîtes de milieu LB, contenant 100 µg. ml⁻¹ d'ampicilline, et en présence d'IPTG et de X-Gal (Sambrook *et al*., 1989).

A l'issue de la transformation, on a isolé un clone qui contient le fragment d'ADNc de 170 pb cloné au site *Sfr*I du vecteur pPCR-Script Amp SK(+) (Stratagene, USA). Ce produit de PCR a ensuite été séquencé selon le protocole « T7 sequencing kit » (Amersham Pharmacia Biotech AB, SE-751 84 Uppsala, Suède), en présence d'alpha-(³⁵S)-dATP. L'analyse de sa séquence montre qu'il est localisé entre les nucléotides 206 et 375 de la séquence SEQ ID NO: 1 et est bordé à ses extrémités 5' et 3' par les séquences correspondant aux oligonucléotides MAN 202 et 203. De cette analyse, on déduit que ce produit de PCR correspond à un fragment de l'ADNc codant effectivement pour l'endo-β-mannanase de café qui a été purifiée et séquencée comme défini précédemment. On constate également que cet ADNc est différent de celui qui a été cloné au laboratoire (EP n° 98203742.6) ce qui suggère l'existence d'un famille multigénique codant pour l'endo-β-mannanase de café.

Pour isoler l'ADNc pleine longueur de l'endo-β-mannanase de café, on réalise une série de réactions de PCR en utilisant cette fois des oligonucléotides de synthèse spécifiques et déduits de la séquence du produit de PCR 170 pb cloné précédemment. Pour cela, on utilise les oligonucléotides MAN 214 , ayant la séquence nucléique SEQ ID NO: 8, et l'oligonucléotide de synthèse MAN 215, ayant la séquence nucléique SEQ ID NO: 9. Les oligonucléotides de synthèse MAN 214 et 215 correspondent respectivement aux nucléotides 307 à 325 et 307 à 290 de la séquence SEQ ID NO: 1 et sont positionnés tête-bêche sur cette même séquence.

Ces réactions de PCR utilisent 10 ng de la banque d'ADNc criblée précédemment, les oligonucléotides MAN 214 et 215 et les oligonucléotides universels T3 et T7 spécifiques du vecteur de clonage pPCR-Script Amp SK(+) (Stratagene, USA) qui correspondent respectivement aux séquences SEQ ID NO: 10 et 11. Ces amorces sont chacune localisés à environ 100 pb de part et d'autre du site de clonage *Sfr*I du vecteur pPCR-Script Amp SK (+). On incube les réactions de PCR selon les conditions décrites précédemment et avec les paramètres suivants: 40 cycles d'amplification (94° C-1 min, 50° C-1 min 30 s, 72° C-3 min) suivis d'une extension finale à 72° C pendant 7 min.

Après migration sur gel d'agarose des produits de PCR, on observe la présence d'un fragment d'ADN d'environ 400 pb amplifié lors de la réaction MAN 215-T3 et d'un fragment d'ADN d'environ 1.2 Kb amplifié lors de la réaction MAN 214-T7. Ces fragments ont été clonés indépendamment dans le vecteur pGEMT-easy (Promega, USA) puis séquencés sur les deux brins (Eurogentec Bel s.a- Parc Scientifique du Sart Tilman - 4102 Seraing-Belgium). L'analyse des séquences nucléiques montre que le produit de PCR amplifié par les oligonucléotides MAN 215-T3 comprend les 307 premiers nucléotides de la séquence SEQ ID NO: 1 alors que le produit de PCR amplifié par les oligonucléotides MAN 214-T7 comprend les 1180 dernières bases de la séquence SEQ ID NO: 1 ainsi qu'une queue de polyA+ de 26 résidus non présentée sur la séquence SEQ ID NO: 1.

Pour isoler l'ADNc pleine longueur de la mannanase de café correspondant à la séquence SEQ ID NO: 1, on a réalisé une dernière réaction de PCR en utilisant 20 ng de la banque d'ADN plasmidique et les oligonucléotides de synthèse MAN 300 et 301. Ces amorces correspondent respectivement aux séquences SEQ ID NO: 12 et 13 et sont localisées respectivement aux extrémités 5' et 3' de la séquence SEQ ID NO: 1. L'amorce MAN 301 a été choisie comme étant localisée juste en amont de la queue de polyA+ présente dans le fragment de PCR amplifié avec les oligonucléotides MAN 214-T7. Cette réaction de PCR a été réalisée dans un volume final de 50 µl contenant 10 ng de plasmide de la banque d'ADNc (JAI=22), 10 mM KCl, 6 mM (NH₄)₂SO₄, 20 mM Tris-HCl, pH 8, 0.1% Triton X-100, 2 mM MgCl₂, 0.2 mM de chaque dNTP, 10 µg/ml BSA, 0.25 µM de chaque oligonucléotide et 3 unités de *Pfu* ADN polymérase (Stratagene, USA). La réaction est incubée pendant 45 cycles (94° C-1 min, 45° C-1 min 30 s, 72° C-3 min) et suivie d'une extension finale à 72° C pendant 7 min.

A l'issue de cette réaction, on amplifie un seul fragment d'environ 1500 pb qui a été cloné dans le vecteur pPCR Script Amp SK (+) puis séquencé sur le deux brins. Sa séquence correspond à la séquence SEQ ID NO: 1. Par comparaison dans les banques de données, on déduit que cet ADNc est pleine longueur et code effectivement pour une endo-β-mannanase qui comprend les séquences protéiques SEQ ID NO: 3 à 5 obtenues précédemment à partir de la purification de l'endo-β-mannanase. On note également que cet ADNc est différent de l'ADNc cloné précédemment au laboratoire (EP no 98203742.6). Il constitue donc un nouvel ADNc codant pour l'endo-β-mannanase de café qui a été purifiée comme décrit précédemment. On note également que cette séquence SEQ ID NO: 1 contient sans aucune différence nucléotidique toutes les séquences des intermédiaires de clonage isolés précédemment lors des amplifications PCR de la banque d'ADNc en cours de germination.

### 4. Analyse de l'ADNc pleine longueur codant pour l'endo-β-mannanase de café

L'analyse de la séquence nucléique montre que cet ADNc pleine longueur contient à son extrémité 5' une séquence transcrite non traduite de 61 pb et une séquence 3' transcrite non traduite de 174 pb. Il ne possède pas de queue de poly A+ à son extrémité 3' puisque l'oligonucléotide de synthèse MAN 301 utilisé précédemment a justement été désigné en amont de cette séquence. Au sein de cette séquence 3' transcrite non traduite, on observe la présence de motifs riches en AT supposés intervenir dans les mécanismes de polyadénylation. On constate aussi la présence de plusieurs séquences répétées directes et inversées qui pourraient intervenir dans des mécanismes de stabilité des ARN messagers ou d'efficacité de la traduction par exemple (Gallie, Plant Mol Biol, 32, 145-158, 1996).

La séquence SEQ ID NO: 1 contient une phase ouverte de lecture de 417 codons, qui commence par le codon ATG en position 62 et se termine par un codon TAA (A en position 1312). On note également la présence d'un deuxième codon d'initiation de la traduction en position 65 de la séquence SEQ ID NO: 1. La protéine déduite de cet ADN complémentaire a un poids moléculaire théorique de 46794 Da et un pI théorique de 7.8. Elle présente également un segment protéique très hydrophobe qui correspond environ aux 30 premiers acides aminés de la séquence SEQ ID NO: 2. Cette séquence protéique pourrait correspondre à un peptide signal contenant les 40 acides aminés de la séquence SEQ ID NO: 1 en amont de la séquence SEQ ID NO: 3 qui correspond au séquençage N-terminal de l'enzyme purifiée. Dans ce cas, on s'attend à ce que le poids moléculaire de la protéine soit de 42616 Da sous sa forme mature c'est à dire correspondant au 376 derniers acides aminés de la séquence SEQ ID NO: 2. Dans ce cas, son pI est estimé proche de 5.8. Ces données sont en accord avec celles observées lors de la purification de la protéine (voir Exemple: 2, § 5).

On note aussi l'existence de plusieurs sites potentiels de glycosylation (Asn / X / Ser ou Thr). Le premier est localisé dans le peptide signal potentiel, en position 35 à 37 de la séquence SEQ ID NO: 2, et est donc supposé être absent dans la forme mature de la mannanase. Le deuxième est localisé en position C-terminale, en position 398 et 400 de la séquence SEQ ID NO: 2.

Au sein de la protéine (SEQ ID NO: 2) déduite de la séquence SEQ ID NO: 1, on note que l'on retrouve sans aucune ambiguïté les séquences SEQ ID NO: 3 à 5 qui correspondent aux séquençages protéiques réalisés à partir de la protéine purifiée. Par exemple, la séquence SEQ ID NO: 3 correspond aux acides aminés 41 à 61 de la SEQ ID NO: 2. De même, on retrouve les séquences SEQ ID NO: 4 et 5 qui correspondent respectivement aux acides aminés 99 à 108 et 265 à 281 de la SEQ ID NO: 2. Les séquences SEQ ID NO: 4 et 5 sont d'ailleurs précédées respectivement par les acides aminés R (position 98 de SEQ ID NO: 2) et K (position 264 de SEQ ID NO: 2) reconnus par la trypsine qui a été utilisée pour effectuer la protéolyse de mannanase purifiée puis le séquençage de certains peptides internes (voir Exemple: 2, § 5).

### 5. Comparaison des séquences protéiques de mannanases de caféier.

L'alignement (Feng and Doolittle, J. Mol. Evol. 25, 351-360, 1987) de la séquence protéique SEQ ID NO: 2, notée mannanase 2, avec la séquence protéique de l'endo-β-mannanase notée mannanase 1, dont l'ADN complémentaire a été cloné précédemment au laboratoire (Marraccini and Rogers, EP n° 98203742.6), montre qu'il existe seulement 55,7% d'identité stricte entre ces deux protéines du caféier (Figure 1). En revanche, il existe plusieurs segments protéiques extrêmement conservés entre ces deux protéines et celle de la tomate (Bewley *et al*., Planta 203: 454-459, 1997) ou d'autres mannanases eucaryotiques comme celles de *Aspergillus aculeatus* (Database accession number: L35487) et *Trichoderma reesei* (L25310). Certaines de ces conservations concernent d'ailleurs des acides aminés supposés être essentiels dans l'activité de la protéine, en particulier les résidus glutamique (E) en position 212, 216, 253 et 333 de la séquence SEQ ID NO: 2 qui pourraient être des résidus catalytiques (Bewley *et al*., 1997). D'autres résidus d'acides aminés comme l'histidine (H) 291, l'asparagine (N) 215, la tyrosine (Y) 293 et le tryptophane (W) 377 de la séquence SEQ ID NO: 2 sont aussi conservés et pourraient être essentiel à la fixation et la dégradation du substrat.

## Revendications

1. Fragment d'ADN issu de café codant pour au moins une enzyme impliquée dans l'hydrolyse de polysaccharides constitués au moins de molécules de mannanes pures ou ramifiée liées entre elles par un liaison β (1->4), présentant la séquence nucléique SEQ ID NO: 1 ou étant homologue ou s'hybridant à un fragment d'ADN présentant la séquence nucléique SEQ ID NO: 1.

2. Fragment d'ADN selon la revendication 1 codant pour au moins une endo-β-mannanase comprenant au moins l'une des séquences suivantes : SEQ ID NO:2, SEQ ID NO:3, SEQ ID NO:4 ou SEQ ID NO:5.

3. Fragment d'ADN selon la revendication 1 comprenant au moins les nucléotides 62 à 1312 de la séquence nucléique SEQ ID NO: 1.

4. Vecteur recombinant comprenant un fragment d'ADN selon l'une des revendications 1 à 5.

5. Utilisation de tout ou partie de fragments d'ADN selon les revendications 1 à 3, d'au moins 10 pb, comme amorce pour faire une PCR ou comme sonde pour détecter *in vitro* ou modifier *in vivo* au moins un gène de café codant pour au moins une endo-β-mannanase.

6. Protéine issue de la graine de café codée par un gène de café et impliquée dans l'hydrolyse de polysaccharides constitués au moins de molécules de mannanes pures ou ramifiés liés entre elles par une liaison β (1->4) et ayant la séquence en acides aminés SEQ ID NO: 2 ou toute séquence en acides aminés homologue à cette dernière, ladite protéine comprenant au moins l'une des séquences suivantes : SEQ ID NO:3, SEQ ID NO:4 ou SEQ ID NO:5.

7. Cellule végétale comprenant, intégré dans son génome ou par le moyen d'un vecteur recombinant, un fragment d'ADN selon l'une des revendications 1 à 3.

8. Cellule végétale selon la revendication 7 **caractérisée par** le fait qu'il s'agit d'une cellule de café.

9. Plante ou graine constituées de cellules végétales selon l'une des revendications 7 et 8.

10. Microorganisme comprenant, intégré dans son génome ou par le moyen d'un plasmide replicable, un fragment d'ADN selon l'une des revendications 1 à 3 de manière à ce qu'il exprime au moins une enzyme impliquée dans l'hydrolyse de polysaccharides constitués au moins de molécules de mannanes pures ou ramifiées liées entre elles par une liaison β (1->4).

11. Composition alimentaire, cosmétique ou pharmaceutique comprenant un fragment d'ADN selon les revendications 1 à 3 ou une protéine selon la revendication 6.

12. Procédé de traitement de grains de café, dans lequel on utilise toute ou partie de la protéine selon la revendication 6.
